# EUROPÄISCHE PATENTANMELDUNG

(11) **EP 4 431 928 A1**
(43) Veröffentlichungstag der Anmeldung: **18.09.2024**
(21) Anmeldenummer: 23162073.3
(22) Anmeldetag: 15.03.2023
(51) Int. Cl.: G01N 24/08, G01R 33/30, G01R 33/422

(54) **MAGNETRESONANZVORRICHTUNG ZUM ERFASSEN ZUMINDEST EINER EIGENSCHAFT EINER PROBE, INSBESONDERE EINES LEBENSMITTELS**

(71) Anmelder: Siemens Healthineers AG, 91301 Forchheim (DE)
(72) Erfinder: Biber, Stephan, 91056 Erlangen (DE); Grodzki, David, 91058 Erlangen (DE)
(74) Vertreter: Siemens Healthineers Patent Attorneys

(57) **Zusammenfassung**

Die Erfindung geht aus von einer Magnetresonanzvorrichtung, insbesondere zum Erfassen von zumindest einer Eigenschaft eines Lebensmittels, umfassend eine Magneteinheit, die einen Grundmagneten und eine Hochfrequenzantenneneinheit umfasst, einen Probenaufnahmebereich, wobei der Probenaufnahmebereich zumindest teilweise von der Magneteinheit umgeben ist, und einer Transportvorrichtung zu einem Einbringen zumindest einer Probe in den Probenaufnahmebereich, wobei die Magnetresonanzvorrichtung eine Hochfrequenzabschirmeinheit aufweist, die den Probenaufnahmebereich nach außen abschirmt.

## Beschreibung

Die vorliegende Erfindung betrifft eine Magnetresonanzvorrichtung, insbesondere zum Erfassen zumindest einer Eigenschaft einer Probe, vorzugsweise eines Lebensmittels, umfassend eine Magneteinheit, die einen Grundmagneten und eine Hochfrequenzeinheit umfasst, einen Probenaufnahmebereich, wobei der Probenaufnahmebereich zumindest teilweise von der Magneteinheit umgeben ist, und eine Transportvorrichtung zu einem Einbringen der Probe in den Probenaufnahmebereich.

Unabhängig vom grammatikalischen Geschlecht eines bestimmten Begriffes sind Personen mit männlicher, weiblicher oder anderer Geschlechteridentität mit umfasst.

Bei der Qualitätskontrolle von Lebensmitteln kann insbesondere die Magnetresonanztechnologie vorteilhaft eingesetzt werden. Beispielsweise können mittels der Magnetresonanztechnologie Fleischprodukte auf Verunreinigungen und/oder hinsichtlich ihres Fettgehalts und/oder hinsichtlich ihrer Qualität untersucht werden. Dabei weist die Magnetresonanztechnologie den Vorteil auf, dass sie keinen direkten Kontakt zum untersuchten Objekt, nichtinvasiv ist und einen guten Weichteilkontrast aufweist.

Zur Verwendung von Magnetresonanzvorrichtungen ist es üblicherweise erforderlich, dass die Magnetresonanzvorrichtung innerhalb einer Hochfrequenz-Kabine (HF-Kabine) und/oder innerhalb eines speziellen HF-geschirmten Raumes angeordnet ist, wie dies beispielsweise bei medizinischen Magnetresonanzvorrichtung üblich ist. Derartige HF-geschirmte Räume und/oder Kabinen sind jedoch sehr aufwendig und teuer.

Der vorliegenden Erfindung liegt insbesondere die Aufgabe zugrunde, eine Magnetresonanzvorrichtung bereitzustellen, die außerhalb eines HF-geschirmten Raums verwendet werden kann. Die Aufgabe wird durch die Merkmale den unabhängigen Anspruch gelöst. Vorteilhafte Ausgestaltungen sind in den Unteransprüchen beschrieben.

Die Erfindung geht aus von einer Magnetresonanzvorrichtung, insbesondere zum Erfassen von zumindest einer Eigenschaft einer Probe, vorzugsweise eines Lebensmittels, umfassend eine Magneteinheit, die einen Grundmagneten und eine Hochfrequenzantenneneinheit umfasst, einen Probenaufnahmebereich, wobei der Probenaufnahmebereich zumindest teilweise von der Magneteinheit umgeben ist, und eine Transportvorrichtung zu einem Einbringen der Probe in den Probeaufnahmebereich. Erfindungsgemäß weist die Magnetresonanzvorrichtung eine Hochfrequenzabschirmeinheit auf, die den Probenaufnahmebereich nach außen abschirmt.

Die Magnetresonanzvorrichtung umfasst bevorzugt eine nichtmedizinische und/oder nicht-diagnostische Magnetresonanzvorrichtung. Bevorzugt ist die Magnetresonanzvorrichtung zu einem Erfassen von Magnetresonanzdaten und/oder Bilddaten ausgebildet, wobei mittels der Magnetresonanzdaten und/oder Bilddaten zumindest eine Eigenschaft der innerhalb des Probenaufnahmebereichs befindlichen Proben, insbesondere von Lebensmitteln, bestimmt werden kann. Beispielsweise kann derart eine Qualität von Fleischprodukten und/oder Verunreinigungen von Fleischprodukten und/oder ein Fettgehalt von Fleischprodukten erfasst und/oder bestimmt werden.

Die Magneteinheit weist bevorzugt einen Grundmagneten, insbesondere einen zylinderförmigen Grundmagneten auf. Der Grundmagnet ist zur Erzeugung eines homogenen Grundmagnetfelds mit einer definierten Magnetfeldstärke, wie beispielsweise mit einer Magnetfeldstärke von 0,55 T oder 1,5 T oder 3 T usw., ausgebildet. Insbesondere ist der Grundmagnet zur Erzeugung eines starken, konstanten und homogenen Grundmagnetfelds ausgebildet. Das homogene Grundmagnetfeld ist bevorzugt innerhalb des Probenaufnahmebereichs der Magnetresonanzvorrichtung angeordnet und/oder vorzufinden. Des Weiteren weist die Magneteinheit die Hochfrequenzantenneneinheit. Die Hochfrequenzantenneneinheit ist zu einem Aussenden von Hochfrequenzpulsen und zu einem Erfassen von Magnetresonanzdaten ausgebildet. Die Hochfrequenzantenneneinheit ist ebenfalls bevorzugt zylinderförmig ausgebildet und innerhalb eines von dem zylinderförmigen Grundmagneten umschlossenen Hohlraum angeordnet.

Zusätzlich kann die Magnetresonanzvorrichtung auch ein Gradientensystem umfassen, das zu einer Erzeugung von Magnetfeldgradienten, die für eine Ortskodierung während einer Bildgebung verwendet werden, ausgebildet ist. Ein derartiges Gradientensystem ist ebenfalls bevorzugt zylinderförmig ausgebildet und innerhalb des von dem zylinderförmigen Grundmagneten umschlossenen Hohlraum angeordnet.

Die Magneteinheit umfasst zudem eine mittige zylinderförmige Aussparung, die den Probenaufnahmebereich umfasst. Dabei ist der Probenaufnahmebereich zylinderförmig von der Magneteinheit, insbesondere von dem Grundmagneten, der Hochfrequenzantenneneinheit und, sofern vorhanden, von dem Gradientensystem umgeben. An einer Frontseite der Magneteinheit weist der Probenaufnahmebereich eine Einführöffnung auf. An einer Heckseite der Magneteinheit weist der Probenaufnahmebereich bevorzugt eine Ausführöffnung auf. Die Einführöffnung ist zu einem Einführen von Proben in den Probenaufnahmebereich ausgebildet. Die Ausführöffnung dagegen ist zu einem Ausführen von Proben aus dem Probenaufnahmebereich ausgebildet, beispielsweise nach einem Beenden der Magnetresonanzmessung an der Probe. Alternativ hierzu kann der Probenaufnahmebereich nur eine einzige Öffnung aufweisen, die eine Funktion der Einführöffnung und der Ausführöffnung aufweist.

Innerhalb des Probenaufnahmebereichs ist bevorzugt ein Field of View (FOV) und/oder ein Isozentrum der Magnetresonanzvorrichtung angeordnet. Das FOV umfasst bevorzugt einen Erfassungsbereich der Magnetresonanzvorrichtung, innerhalb dessen die Bedingungen für eine Erfassung Magnetresonanzdaten vorliegen, wie beispielsweise ein homogenes Grundmagnetfeld. Das Isozentrum der Magnetresonanzvorrichtung umfasst bevorzugt den Bereich und/oder Punkt innerhalb der Magnetresonanzvorrichtung, der die optimalen und/oder idealen Bedingungen für die Erfassung von Magnetresonanzdaten aufweist. Insbesondere umfasst das Isozentrum den homogensten Magnetfeldbereich innerhalb der Magnetresonanzvorrichtung.

Die Transportvorrichtung ist zu einem Einbringen von Proben in den Probenaufnahmebereich ausgebildet. Insbesondere ist die Transportvorrichtung zu einem kontinuierlichen und/oder fortlaufenden Einbringen von Proben in den Probenaufnahmebereich ausgebildet. Des Weiteren ist die Transportvorrichtung nach der Magnetresonanzmessung an der Probe auch zu einem Hinausbringen der Probe aus dem Probenaufnahmebereich ausgebildet. Bevorzugt umfasst hierbei die Transportvorrichtung ein Transportband mit einer Transportrichtung, wobei das Transportband in Transportrichtung Proben über die Einführöffnung in den Probenaufnahmebereich eingeführt und über die Ausführöffnung aus den Patientenaufnahmebereich ausführt. Dabei können mehrere Proben gleichzeitig auf dem Transportband positioniert und/oder gelagert sein, wobei die einzelnen Proben bevorzugt einen definierten Abstand zueinander aufweisen. Beispielsweise kann der definierte Abstand zwischen den einzelnen Proben derart dimensioniert sein, dass jeweils nur eine einzige Probe während einer Magnetresonanzmessung innerhalb des FOVs angeordnet ist.

Zu einer Abschirmung von Hochfrequenzstrahlung weist die Magnetresonanzvorrichtung die Hochfrequenzabschirmeinheit auf. Die Hochfrequenzabschirmeinheit schirmt dabei den Probenaufnahmebereich nach außen hinsichtlich einer Hochfrequenzstrahlung ab. Vorzugsweise weisen dabei einzelnen Bauteile der Hochfrequenzabschirmeinheit einen elektrischen Kontakt zueinander auf, so dass das Innere des Probenaufnahmebereichs nach außen abgeschirmt ist. Beispielsweise kann mittels er Hochfrequenzabschirmeinheit im Bereich der Larmorfrequenz eine Abschirmung und/oder eine Dämpfung von 40 dB bis 100 dB erreicht werden. Zudem kann der elektrische Kontakt der Hochfrequenzabschirmeinheit auch zu einem Metallgehäuse der Magneteinheit geschlossen werden.

Die vorliegende Erfindung weist den Vorteil auf, dass eine einfache und kostengünstige Hochfrequenzabschirmung bereitgestellt werden kann, die bereits von der Magnetresonanzvorrichtung umfasst ist. Derart kann auf einen zusätzliche HF-Kabine und/oder einen zusätzlichen geschirmten Raum, innerhalb dem die Magnetresonanzvorrichtung angeordnet wird, verzichtet werden. Dies ermöglicht auch eine einfache Installation einer erfindungsgemäßen Magnetresonanzvorrichtung, auch in einem nicht HF-geschirmten Umfeld.

Ein weiterer Vorteil der Erfindung ist, dass eine einfache und insbesondere strahlungsfreie Erfassung von Probeneigenschaften, insbesondere von Eigenschaften von Lebensmitteln, beispielsweise zur Qualitätskontrolle von Lebensmitteln, bereitgestellt werden kann. Zudem kann die Erfassung von Probeneigenschaften von insbesondere Lebensmitteln kontaktlos zur Probe, insbesondere dem Lebensmittel, erfolgen, so dass die Erfassung, beispielsweise bei einer Qualitätskontrolle, auch mit einem geringeren Reinigungsaufwand verbunden ist und/oder bei der Einhaltung von Hygienevorschriften vereinfacht werden kann. Zudem können mittels der Magnetresonanztomographie auch Daten mit einem hohen Weichteilkontrast bereitgestellt werden, so dass vorteilhaft Eigenschaften, wie beispielsweise ein Fettanteil und/oder eine Qualität in Fleischprodukten ermittelt werden kann.

In einer vorteilhaften Weiterbildung der erfindungsgemäßen Magnetresonanzvorrichtung kann es vorgesehen sein, dass der Probenaufnahmebereich zylinderförmig von der Magneteinheit umgeben ist, eine Einführöffnung und eine Ausführöffnung aufweist, wobei die Hochfrequenzabschirmeinheit um die Einführöffnung und/oder um die Ausführöffnung angeordnet ist. Vorzugsweise ist die Einführöffnung des Patientenaufnahmebereichs an einer Frontseite der Magneteinheit angeordnet und die Ausführöffnung des Patientenaufnahmebereichs an einer Heckseite der Magneteinheit angeordnet. Durch die Anordnung der Hochfrequenzabschirmeinheit kann eine vorteilhafte HF-Abschirmung der Probenaufnahmebereichs erreicht werden. Insbesondere können derart unerwünschte Beeinträchtigungen, beispielsweise der Erfassung einer Probeneigenschaft mittels der Magnetresonanzvorrichtung oder auch eine Beeinträchtigung von außerhalb der Magnetresonanzvorrichtung befindlichen Geräten, verhindert werden.

In einer vorteilhaften Weiterbildung der erfindungsgemäßen Magnetresonanzvorrichtung kann es vorgesehen sein, dass die Hochfrequenzabschirmeinheit außerhalb des Probenaufnahmebereichs angeordnet ist. Diese Ausgestaltung der Erfindung weist den Vorteil auf, dass der Probenaufnahmebereich nicht durch die Hochfrequenzabschirmeinheit eingeschränkt wird und ein großer Probenaufnahmebereich für Magnetresonanzmessungen von Proben beibehalten werden kann. Zudem kann eine Anordnung und/oder Positionierung der Hochfrequenzabschirmeinheit konstruktiv einfach an der Frontseite und/oder an der Heckseite der Magneteinheit angeordnet werden und verursacht keine Störungen innerhalb des Probenaufnahmebereichs und/oder während einer Magnetresonanzmessung.

In einer vorteilhaften Weiterbildung der erfindungsgemäßen Magnetresonanzvorrichtung kann es vorgesehen sein, dass die Hochfrequenzabschirmeinheit zumindest eine Türeinheit aufweist, wobei mittels der zumindest einen Türeinheit der Probenaufnahmebereich verschließbar oder öffenbar ausgebildet ist. Vorzugsweise umfasst die zumindest eine Türeinheit zumindest eine Tür und/oder eine Klappe, wobei die zumindest eine Tür und/oder die zumindest eine Klappe zusätzlich als HF-Schirmelement ausgebildet ist. Dabei kann die zumindest eine Tür und/oder die zumindest eine Klappe beispielsweise elektrisch leitend ausgebildet sein oder einen elektrischen Kontakt zu einem Gehäuse der Magneteinheit aufweisen, beispielsweise bei einer Ausbildung der zumindest einen Tür und/oder der zumindest einen Klappe aus einem Metall. Insbesondere kann mittels der zumindest eine Türeinheit der Probenaufnahmebereich für ein Einführen einer Probe in den Probenaufnahmebereich und/oder für Herausführen einer Probe aus dem Probenaufnahmebereich geöffnet werden. Dagegen ist der Probenaufnahmebereich während einer Magnetresonanzmessung mittels der zumindest einen Türeinheit geschlossen, insbesondere HF-dicht verschlossen. Vorzugsweise weist die Magneteinheit zwei Türeinheiten auf, wobei eine erste Türeinheit vor der Einführöffnung des Probenaufnahmebereichs angeordnet ist und eine zweite Türeinheit nach der Ausführöffnung des Probenaufnahmebereichs angeordnet ist. Alternativ kann die Hochfrequenzabschirmeinheit auch nur eine einzige Türeinheit umfassen, wobei die Einführöffnung bei einer derartigen Ausbildung auch die Funktion einer Ausführöffnung aufweist, und der Probenaufnahmebereich an der Heckseite fest verschlossen, insbesondere HF-dicht verschlossen, ausgebildet ist.

Diese Ausgestaltung der Erfindung weist den Vorteil auf, dass eine einfach HF-Schirmung während einer Magnetresonanzmessung erreicht werden kann und gleichzeitig eine gute Zugänglichkeit zu dem Probenaufnahmebereich zwischen den einzelnen Magnetresonanzmessungen erhalten bleibt.

In einer vorteilhaften Weiterbildung der erfindungsgemäßen Magnetresonanzvorrichtung kann es vorgesehen sein, dass die Hochfrequenzabschirmeinheit zumindest ein zylinderförmiges Abstandselement aufweist, das an einer Frontseite und/oder an einer Heckseite der Magneteinheit angeordnet ist, wobei das zumindest eine Abstandselement zwischen dem Probenaufnahmebereich und der zumindest einen Türeinheit angeordnet ist. Bevorzugt ist das zumindest eine zylinderförmige Abstandselement als HF-Schirmelement ausgebildet, beispielsweise als Metallzylinder ausgebildet. Das zylinderförmige Abstandselement kann dabei nur wenige cm lang sein. Zudem kann das zylinderförmige Abstandselement auch bis zu 200 cm oder mehr lang sein und somit an unterschiedliche Platzverhältnisse angepasst werden. Bevorzugt ist das zumindest eine zylinderförmige Abstandselement an einem Gehäuse der Magneteinheit, insbesondere einer Frontseite und/oder einer Heckseite der Magneteinheit, angeordnet. Diese Ausgestaltung der Erfindung weist den Vorteil auf, dass die zumindest einen Türeinheit in einem Bereich mit einem geringen Wechselfeld angeordnet werden kann und damit unerwünschte Störungen des Magnetfelds und/oder unerwünschte Wirbelströme verhindert werden können.

In einer vorteilhaften Weiterbildung der erfindungsgemäßen Magnetresonanzvorrichtung kann es vorgesehen sein, dass zumindest eine zylinderförmige Abstandselement HF-dicht an der Frontseite und/oder an der Heckseite der Magneteinheit angeordnet ist. Für die HF-dichte Anordnung des zumindest einen zylinderförmigen Abstandselement kann das zumindest eine zylinderförmige Abstandselement mit einem Gehäuse der Magneteinheit, insbesondere an einer Frontgehäuseseite und/oder an einer Heckgehäuseseite der Magneteinheit, verschweißt sein. Des Weiteren kann das zumindest eine zylinderförmige Abstandselement auch an das Gehäuse der Magneteinheit, insbesondere an einer Frontgehäuseseite und/oder an einer Heckgehäuseseite der Magneteinheit, geklemmt werden. Für eine HF-dichte Anordnung des zumindest einen zylinderförmigen Abstandselements können dabei beispielsweise HF-dichte Kontaktfedern zwischen dem zumindest einen zylinderförmigen Abstandselement und der Gehäuseeinheit der Magneteinheit, insbesondere der Frontgehäuseseite und/oder der Heckgehäuseseite der Magneteinheit, angeordnet sein. Bevorzugt sind derartige Kontaktfedern aus Metall oder elektrisch leitend ausgebildet, so dass ein elektrischer Kontakt zwischen der Gehäuseeinheit der Magneteinheit, insbesondere der Frontgehäuseseite und/oder der Heckgehäuseseite der Magneteinheit, und dem zumindest einem zylinderförmigen Abstandselement für eine HF-Schirmung bereitsteht. Beispielsweise können diese Kontaktfedern, insbesondere die elektrisch leitenden Kontaktfedern, eine lamellenartige Struktur aufweisen. Derart kann besonders einfach und kostengünstig eine Hochfrequenzabschirmung durch die Anordnung des zylinderförmige Abstandselements erreicht und/oder aufrechterhalten werden.

In einer vorteilhaften Weiterbildung der erfindungsgemäßen Magnetresonanzvorrichtung kann es vorgesehen sein, dass die zumindest eine Türeinheit zumindest eine Klappe aufweist und die Hochfrequenzabschirmeinheit zumindest eine elektrische Antriebseinheit und/oder zumindest eine pneumatische Antriebseinheit aufweist, wobei mittels der zumindest einen elektrischen Antriebseinheit und/oder der zumindest einen pneumatischen Antriebseinheit ein Antriebsmoment für eine Bewegung der zumindest einen Klappe der zumindest einen Türeinheit generierbar ist. Die zumindest eine Klappe der zumindest einen Türeinheit ist zu einem Öffnen oder einem Verschließen des Probenaufnahmebereichs ausgebildet. Bevorzugt ist die zumindest eine Klappe an einer der Magneteinheit abgewandten Seite des zumindest einen zylinderförmigen Abstandselements angeordnet. Die zumindest eine Klappe kann zum Öffnen des Probenaufnahmebereichs und/oder zum Schließen des Probenaufnahmebereichs eine Linearbewegung oder auch eine Drehbewegung ausführen.

Bevorzugt sind die zumindest eine elektrische Antriebseinheit und/oder die zumindest eine pneumatische Antriebseinheit außerhalb des Probenaufnahmebereichs und außerhalb des von der Hochfrequenzabschirmeinheit umschlossenen und/oder umschließbaren Bereichs angeordnet. Die zumindest eine elektrische Antriebseinheit kann dabei einen Elektromotor aufweisen. Derart kann eine einfache und kostengünstige Antriebseinheit zu einem Öffnen und/oder Verschließen der zumindest einen Türeinheit bereitgestellt werden. Zudem kann mittels der pneumatischen Antriebseinheit eine unerwünschte Störung und/oder Interaktion mit dem Hochfrequenzfeld der Magneteinheit verhindert werden. Zudem kann mittels zumindest einen zylinderförmigen Abstandselements die Antriebseinheit, insbesondere die elektrische Antriebseinheit und/oder die pneumatische Antriebseinheit beabstandet zur Magneteinheit angeordnet werden und damit in einem Bereich mit einem geringen magnetischen Feld und/oder einem geringen Hochfrequenzfeld angeordnet werden.

In einer vorteilhaften Weiterbildung der erfindungsgemäßen Magnetresonanzvorrichtung kann es vorgesehen sein, dass die Hochfrequenzabschirmeinheit zumindest ein Übertragungselement aufweist, um ein von der zumindest einen elektrischen Antriebseinheit und/oder der zumindest einen pneumatischen Antriebseinheit generiertes Antriebsmoment auf die zumindest eine Klappe der zumindest einen Türeinheit zu übertragen. Umfasst die Hochfrequenzabschirmeinheit eine elektrische Antriebseinheit, ist das zumindest eine Übertragungselement bevorzugt von einem Übertragungsriemen und/oder einer Kette gebildet. Umfasst dagegen die Hochfrequenzabschirmeinheit eine pneumatische Antriebseinheit, umfasst das zumindest eine Übertragungselement beispielsweise einen Luftschlauch und/oder eine pneumatische Leitung. Derart kann vorteilhaft die Antriebseinheit, insbesondere die elektrische Antriebseinheit und/oder auch die pneumatische Antriebseinheit, in einem großen Abstand zur Magneteinheit, insbesondere zum Probenaufnahmebereich der Magneteinheit, angeordnet werden, beispielsweise einen Abstand von zu 3 m zur Magneteinheit. So können auch unerwünschte Interaktionen und/oder Beeinträchtigungen zwischen der Antriebseinheit, insbesondere der elektrischen Antriebseinheit, und der HF-Feld der Magneteinheit vorteilhaft verhindert werden. Insbesondere kann derart die elektrische Antriebseinheit vor unerwünschten Störungen und/oder Beeinträchtigung ihrer Funktionalität geschützt werden.

In einer vorteilhaften Weiterbildung der erfindungsgemäßen Magnetresonanzvorrichtung kann es vorgesehen sein, dass die zumindest eine Türeinheit eine erste Klappe und eine zweite Klappe umfasst, wobei die erste Klappe und die zweite Klappe HF-dicht verschließbar sind. Derart kann ein einfaches Öffnen und Schließen des Probenaufnahmebereichs erreicht werden. Zudem kann derart die einzelne Klappe kleiner ausgeführt werden, wodurch ein auf den Schließmechanismus lastendes Gewicht reduziert werden kann und damit ein Verschleiß reduziert werden kann.

In einer vorteilhaften Weiterbildung der erfindungsgemäßen Magnetresonanzvorrichtung kann es vorgesehen sein, dass die zumindest eine Türeinheit zumindest eine HF-Dichtung aufweist, wobei die HF-Dichtung in einem Kontaktbereich zwischen der ersten Klappe und der zweiten Klappe und/oder in einem Kontaktbereich zwischen der ersten Klappe und dem zumindest einen zylinderförmigen Abstandelement und/oder in einem Kontaktbereich zwischen der zweiten Klappe und dem zumindest einen zylinderförmigen Abstandselement angeordnet ist. Vorzugsweise weist die zumindest eine Türeinheit mehrere HF-Dichtungen auf, die in allen Kontaktbereich der ersten Klappe mit der zweiten Klappe und in allen Kontaktbereichen der ersten Klappe und der zweiten Klappe mit dem zumindest einen zylinderförmigen Abstandselement angeordnet sind. Die einzelnen HF-Dichtungen sind bevorzugt von HF-dichten Kontaktfedern gebildet. Bevorzugt sind derartige HF-dichte Kontaktfedern aus Metall oder elektrisch leitend ausgebildet, so dass in einem geschlossenen Zustand der zumindest einen Türeinheit ein elektrischer Kontakt zwischen den beiden Klappen und/oder zwischen der ersten Klappe und dem zumindest einen zylinderförmigen Abstandselement und/oder der zweiten Klappe und dem zumindest einen zylinderförmigen Abstandselement besteht. Beispielsweise können diese Kontaktfedern, insbesondere die elektrisch leitenden Kontaktfedern, eine lamellenartige Struktur aufweisen. Derart kann besonders einfach und kostengünstig eine effektive Hochfrequenzabschirmung zwischen allen beweglichen Bauteilen und/oder an allen Kontaktflächen der beweglichen Bauteile der zumindest einen Türeinheit erreicht werden.

In einer vorteilhaften Weiterbildung der erfindungsgemäßen Magnetresonanzvorrichtung kann es vorgesehen sein, dass die Transportvorrichtung zumindest ein Transportband aufweist, wobei das zumindest eine Transportband zumindest eine Aussparung aufweist, wobei die erste Klappe und/oder die zweite Klappe in einem geschlossenen Zustand der zumindest einen Türeinheit zumindest teilweise innerhalb der zumindest einen Aussparung angeordnet sind. Die zumindest eine Aussparung kann dabei ein Längserstreckung aufweisen, die parallel zu einer Querrichtung des Transportband ausgerichtet ist. Vorzugsweise weist das zumindest eine Transportband mehrere Aussparungen auf die Querrichtung des Transportbands nacheinander angeordnet sind. Der geschlossene Zustand der zumindest einen Türeinheit umfasst bevorzugt eine Schließen der beiden Klappen, so dass jede dieser beiden Klappen einen Kontakt mit der anderen der beiden Klappe aufweist. Der Kontakt zwischen den beiden Klappen der zumindest einen Türeinheit kann dabei einen mechanischen und besonders vorteilhaft auch einen elektrischen Kontakt zwischen der ersten Klappe und der zweiten Klappe umfassen. Vorzugsweise ist ein Kontaktbereich der ersten Klappe mit der zweiten Klappe und/oder ein Kontaktbereich der zweiten Klappe mit der ersten Klappe in einem geschlossenen Zustand der zumindest einen Türeinheit innerhalb der zumindest einen Aussparung angeordnet. Derart können die erste Klappe zusammen mit der zweiten Klappe durch die zumindest eine Aussparung in dem Transportband bei einem Schließen der beiden Klappen eine Kontakt, insbesondere einen elektrischen Kontakt, zueinander herstellen und damit eine Hochfrequenzabschirmung in diesem geschlossenen Zustand der zumindest einen Türeinheit erzeugen.

In einer vorteilhaften Weiterbildung der erfindungsgemäßen Magnetresonanzvorrichtung kann es vorgesehen sein, dass das zumindest eine Transportband mehrere Aussparungen aufweist, die in Querrichtung des Transportbands in einer Linie nacheinander angeordnet sind, wobei die erste Klappe in einem Kontaktbereich mit der zweiten Klappe eine Verzahnung aufweist und die zweite Klappe in einem Kontaktbereich mit der ersten Klappe eine zur ersten Klappe korrespondierende Verzahnung aufweist, wobei in einem geschlossenem Zustand der Türeinheit die beiden Verzahnungen durch die Aussparungen des Transportbands ineinander greifen. Vorzugsweise sind hierbei die in Querrichtung des Transportbands verlaufenden Aussparungen, insbesondere eine Form und/oder Kontur der Aussparungen, auf die Verzahnung der ersten Klappe und auf die Verzahnung der zweiten Klappe abgestimmt und/oder korrespondierend zur Verzahnung der ersten Klappe und der zweiten Klappe ausgebildet. Die Querrichtung des Transportbands erstreckt sich bevorzugt quer zur Laufrichtung und/oder Transportrichtung des Transportbands. Durch die mehreren Aussparungen in Querrichtung des Transportbands kann zum einen ein vorteilhaftes Ineinandergreifen der beiden Klappen gewährleistet werden und zum anderen auch eine Stabilität des Transportbands erhalten bleiben.

In einer vorteilhaften Weiterbildung der erfindungsgemäßen Magnetresonanzvorrichtung kann es vorgesehen sein, dass das zumindest eine Transportband mehrere in Längsrichtung nacheinander angeordnete Aussparungen aufweist. Die Längsrichtung des zumindest einen Transportbands erstreckt sich vorzugsweise in Transportrichtung und/oder Bewegungsrichtung des Transportbands. Die Aussparungen sind bevorzugt in gleichmäßigen Abständen nacheinander in Längsrichtung an dem Transportband angeordnet. Dabei kann auch ein Abstand zwischen den einzelnen Aussparungen in Längsrichtung an die zu untersuchenden Proben angepasst sein. Beispielsweise kann zwischen zwei in Längsrichtung angeordneten Aussparungen ein Lagerbereich zur Lagerung und/oder Positionierung einer Probe angeordnet sein. Diese Ausgestaltung der Erfindung weist den Vorteil auf, dass eine hohe Effizienz bei einem Einführen der Proben, beispielsweise von Lebensmitteln, in den Probenaufnahmebereich erreicht werden kann. Beispielsweise können hierbei die Proben mit einem geringen Abstand zueinander auf dem Transportband positioniert werden und trotzdem bei jeder Magnetresonanzmessung einer Probe die zumindest eine Türeinheit HF-dicht geschlossen werden.

In einer vorteilhaften Weiterbildung der erfindungsgemäßen Magnetresonanzvorrichtung kann es vorgesehen sein, dass die Transportvorrichtung zumindest zwei Transportbänder, die nacheinander in Transportrichtung angeordnet sind, und zumindest einen Übergangsbereich zwischen den beiden Transportbändern aufweist, wobei in diesem zumindest einen Übergangsbereich die zumindest eine Türeinheit in einem geschlossenen Zustand der zumindest einen Türeinheit angeordnet ist. Beispielsweise kann die Transportvorrichtung drei Transportbänder umfassen, wobei ein erstes Transportband innerhalb des Probenaufnahmebereichs und innerhalb des von der Hochfrequenzabschirmeinheit umschlossenen Bereichs angeordnet ist. Ein zweites und ein drittes Transportband befinden sich dagegen außerhalb des von der Hochfrequenzabschirmeinheit umschlossenen Bereichs und sind in Transportrichtung vor der Magneteinheit bzw. nach der Magneteinheit angeordnet. Vorzugsweise weist der Übergangsbereich in Transportrichtung eine Länge auf, die ein Schließen der Türeinheit, insbesondere der beiden Klappen der Türeinheit, ermöglicht und zudem in geöffneten Zustand der Türeinheit eine Probenübergabe zwischen den beiden Transportbändern gewährleistet. Diese Ausgestaltung der Erfindung ermöglicht ein einfaches und sicheres Schließen der zumindest einen Türeinheit. Insbesondere kann derart eine sichere Hochfrequenzabschirmung während einer Magnetresonanzmessung erreicht werden.

In einer vorteilhaften Weiterbildung der erfindungsgemäßen Magnetresonanzvorrichtung kann es vorgesehen sein, dass die Transportvorrichtung zumindest ein Transportband aufweist, wobei das zumindest ein Transportband ein HF-schirmendes Material umfasst. Vorzugsweise umfasst hierbei das zumindest ein Transportband HF-schirmende Segmente. Vorzugsweise umfasst das Transportband, das außerhalb des Probenaufnahmebereichs angeordnet ist, das HF-schirmendes Material und/oder die HF-schirmende Segmente. Das HF-schirmende Material umfasst bevorzugt ein elektrisch leitendes Material, insbesondere in Metall. Dagegen ist derjenige Teilbereich der Transportvorrichtung, insbesondere das innerhalb des Probenaufnahmebereichs angeordnete Transportband, aus einem nichtleitenden Material gebildet, um eine unerwünschte Anziehung durch den Grundmagneten der Magneteinheit und/oder eine Erzeugung von Wirbelströmen zu verhindern. Das HF-schirmende Material erzeugt bevorzugt bei einem Schließen der zumindest einen Türeinheit, insbesondere der beiden Klappen der zumindest einen Türeinheit, dass die beiden Klappen der zumindest einen Türeinheit miteinander in Kontakt, insbesondere in einen elektrischen Kontakt, kommen können und damit eine geschlossene Hochfrequenzabschirmung erzeugt wird.

In einer vorteilhaften Weiterbildung der erfindungsgemäßen Magnetresonanzvorrichtung kann es vorgesehen sein, dass die Magnetresonanzvorrichtung eine Steuereinheit aufweist, wobei die Steuereinheit dazu ausgebildet ist, eine Bewegung der Transportvorrichtung und/oder ein Schließen der zumindest einen Türeinheit automatisch an Magnetresonanzmessungen von aufeinander folgenden Proben anzupassen. Derart kann vorteilhaft eine zeitsparende und automatisierte und/oder selbsttätige Erfassung von Probeneigenschaften bereitgestellt werden.

Die erfindungsgemäße Steuereinheit umfasst zumindest ein Rechenmodul und/oder einen Prozessor. So ist insbesondere die Steuereinheit dazu ausgebildet, computerlesbare Instruktionen auszuführen. Insbesondere umfasst die Steuereinheit eine Speichereinheit, wobei auf der Speichereinheit computerlesbare Informationen gespeichert sind, wobei die Steuereinheit dazu ausgebildet ist, die computerlesbaren Informationen von der Speichereinheit zu laden und die computerlesbaren Informationen auszuführen. Derart ist die erfindungsgemäße Steuereinheit dazu ausgebildet, eine Bewegung der Transportvorrichtung und/oder ein Schließen der zumindest einen Türeinheit automatisch an Magnetresonanzmessungen von aufeinander folgenden Proben anzupassen.

Die Komponenten der Steuereinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Weitere Vorteile, Merkmale und Einzelheiten der Erfindung ergeben sich aus des im Folgenden beschriebenen Ausführungsbeispielen sowie anhand der Zeichnungen.

Es zeigen:
- Fig. 1: ein ersten Ausführungsbespiel der Magnetresonanzvorrichtung mit einer Hochfrequenzabschirmung in einer schematischen Darstellung,
- Fig. 2: eine Draufsicht auf eine Transportband der Magnetresonanzvorrichtung,
- Fig. 3: ein zweites Ausführungsbespiel der Magnetresonanzvorrichtung mit einer Hochfrequenzabschirmung in einer schematischen Darstellung und
- Fig. 4: ein drittes Ausführungsbespiel der Magnetresonanzvorrichtung mit einer Hochfrequenzabschirmung in einer schematischen Darstellung.

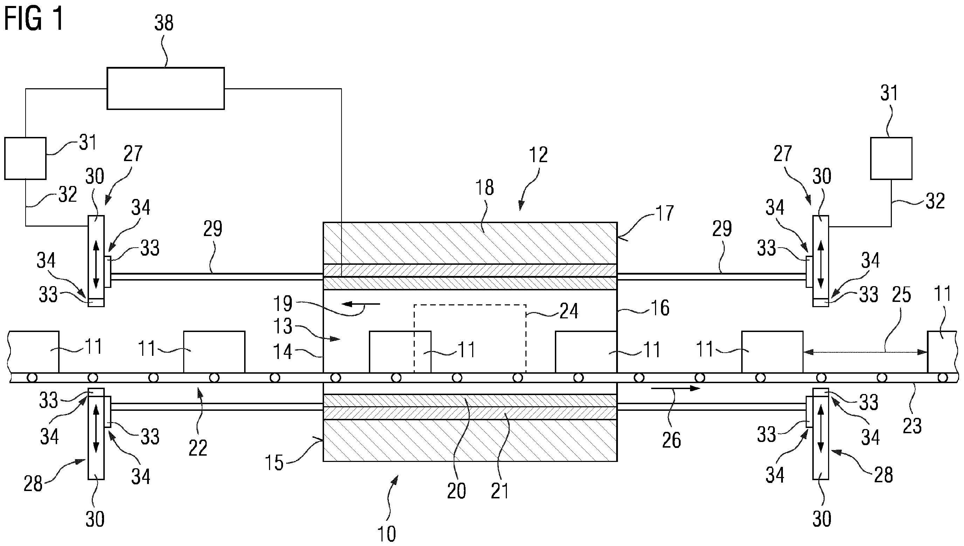

In der Fig. 1 ist ein erstes Ausführungsbeispiel einer Magnetresonanzvorrichtung 10 schematisch dargestellt. Die Magnetresonanzvorrichtung 10 ist insbesondere zu einem Erfassen von zumindest einer Eigenschaft einer Probe 11, insbesondere eines Lebensmittels, ausgebildet. Beispielsweise wird mittels der erfindungsgemäßen Magnetresonanzvorrichtung 10 eine Qualität von Fleischprodukten und/oder ein Fettanteil von Fleischprodukten und/oder unerwünschte Einschlüsse in Fleischprodukten ermittelt und/oder erfasst.

Die Magnetresonanzvorrichtung 10 umfasst eine Magneteinheit 12 und einen Probenaufnahmebereich 13. Der Probenaufnahmebereich 13 erstreckt sich zylinderförmig durch die Magneteinheit 12 hindurch, so dass die Magneteinheit 12 den Probenaufnahmebereich 13 zylinderförmig umgibt. Der Probenaufnahmebereich 13 weist zudem eine Einführöffnung 14, die an einer Frontseite 15 der Magneteinheit 10 angeordnet ist, und eine Ausführöffnung 16, die an einer Heckseite 17 der Magneteinheit 12 angeordnet ist, auf. Grundsätzlich ist jedoch eine davon abweichende Ausbildung des Probenaufnahmebereichs 13 jederzeit denkbar, wie beispielsweise ein Probenaufnahmebereich 13 mit nur einer Öffnung, die zugleich die Funktion einer Einführöffnung und einer Ausführöffnung aufweist.

Die Magneteinheit 12 weist einen supraleitenden Grundmagneten 18 auf, der zu einer Erzeugung eines homogenen Grundmagnetfelds 19 ausgebildet ist. Des Weiteren weist die Magneteinheit 12 eine Hochfrequenzantenneneinheit 20 auf, die zu einem Aussenden von Hochfrequenzpulsen und zu einem Erfassen von Magnetresonanzsignalen ausgebildet ist. Aufgrund der in den Probenaufnahmebereich 13 eingestrahlten Hochfrequenzpulse, die von der Hochfrequenzantenneneinheit 20 eingestrahlt werden, werden in den einzelnen Proben Magnetresonanzsignale hervorgerufen, die wiederum mittels der Hochfrequenzantenneneinheit 20 empfangen werden. Die Hochfrequenzantenneneinheit 20 weist hierzu einen Sendemodus und einen Empfangsmodus auf. Im vorliegenden Ausführungsbeispiel weist die Magneteinheit 12 zudem eine Gradientenspuleneinheit 21 auf, die zu einer Erzeugung von Magnetfeldgradienten auf, die für eine Ortskodierung während einer Magnetresonanzmessung verwendet wird.

Zu einem Einbringen von Proben 11 und/oder Objekten in den Probenaufnahmebereich 13 weist die Magnetresonanzvorrichtung 10 eine Transportvorrichtung 22 auf. Die Transportvorrichtung 22 umfasst im vorliegenden Ausführungsbeispiel ein einziges Transportband 23, auf dem die einzelnen Proben 11 in den Probenaufnahmebereich 13, insbesondere in ein FOV 24 und/oder ein Isozentrum der Magneteinheit 12 für eine Magnetresonanzmessung eingebracht werden. Im vorliegenden Ausführungsbeispiel weisen die einzelnen Proben 11 einen definierten Abstand 25 auf dem Transportband 23 zueinander auf, so dass immer nur eine einzige Probe 11 für eine Magnetresonanzmessung im FOV 24 und/oder im Isozentrum der Magneteinheit 12 sich befindet. Das Transportband 23 weist eine Transportrichtung 26 auf, die sich in Längserstreckung des Transportbands 23 erstreckt. Zudem weist das Transportband 23 eine wesentlich längere Erstreckung auf als der Probenaufnahmebereich 13, so dass die einzelnen Proben 11 nacheinander fortlaufend von au-ßen in den Probenaufnahmebereich 13 eingefahren werden, dort im FOV 24 und/oder im Isozentrum der Magneteinheit 12 für die Magnetresonanzmessung kurz verweilen und anschließend in Transportrichtung 26 wieder den Probenaufnahmebereich 23 über die Ausführöffnung 16 verlassen.

Die Magnetresonanzvorrichtung 10 weist zudem eine Hochfrequenzabschirmeinheit 27 auf, die den Probenaufnahmebereich 13 nach außen abschirmt. Die Hochfrequenzabschirmeinheit 27 ist dabei außerhalb des Probenaufnahmebereichs 13 angeordnet. Im vorliegenden Ausführungsbeispiel ist die Hochfrequenzabschirmeinheit 27 sowohl um die Einführöffnung 14 des Probenaufnahmebereichs 13 als auch um die Ausführöffnung 16 des Probenaufnahmebereichs 13 angeordnet.

Zur Hochfrequenzabschirmung des Probenaufnahmebereichs 13 weist die Hochfrequenzabschirmeinheit 27 zwei Türeinheiten 28 auf. Eine erste Türeinheit 28 ist dabei in einem einer Frontseite 15 der Magneteinheit 12 zugewandten Bereich angeordnet. Eine zweite Türeinheit 28 ist dabei in einem einer Heckseite 17 der Magneteinheit 12 zugewandten Bereich angeordnet. Die beiden Türeinheiten 28 sind jedoch beabstandet zur Magneteinheit 12 angeordnet. Hierzu weist die Hochfrequenzabschirmeinheit 27 zwei zylinderförmige Abstandselemente 29 auf, die zwischen der Magneteinheit 12, insbesondere dem Probenaufnahmebereich 13, und den beiden Türeinheiten 28 angeordnet sind. Dabei ist ein erstes zylinderförmiges Abstandselement 29 an der Frontseite 15 der Magneteinheit 12 und ein zweites zylinderförmiges Abstandselement 29 an der Heckseite 17 der Magneteinheit 12 angeordnet. Zudem sind die beiden Türeinheiten 27 an jeweils einer der Magneteinheit 12 abgewandten Seite der zylinderförmigen Abstandselemente 29 angeordnet. Die beiden zylinderförmigen Abstandselemente 29 können dabei nur wenige cm lang sein oder auch bis zu 200 cm lang sein. Bevorzugt sind die beiden zylinderförmigen Abstandselemente 29 zwischen 100 cm und 200 cm lang. Bevorzugt sind die beiden zylinderförmigen Abstandselemente 29 zwischen 120 cm und 200 cm lang. Bevorzugt sind die beiden zylinderförmigen Abstandselemente 29 zwischen 140 cm und 200 cm lang. Bevorzugt sind die beiden zylinderförmigen Abstandselemente 29 zwischen 160 cm und 200 cm lang. Bevorzugt sind die beiden zylinderförmigen Abstandselemente 29 zwischen 180 cm und 200 cm lang.

Das erste zylinderförmige Abstandselement 29 ist um die Einführöffnung 13 an der Frontseite 15 der Magneteinheit 12 angeordnet. Insbesondere ist das erste zylinderförmige Abstandselement 29 an einem Frontgehäuse der Magneteinheit 12 angeordnet. Im vorliegenden Ausführungsbeispiel ist das erste zylinderförmige Abstandselement 29 mit der Frontseite 15 und/oder dem Frontgehäuse der Magneteinheit 12 verschweißt. Alternativ hierzu kann das erste zylinderförmige Abstandselement 29 auch mit der Frontseite 15 und/oder dem Frontgehäuse der Magneteinheit 12 verklemmt sein und ein Kontaktbereich zwischen dem ersten zylinderförmige Abstandselement 29 und der Frontseite 15 und/oder dem Frontgehäuse der Magneteinheit 12 mittels HF-dichter Kontaktfedern HF-dicht gehalten werden.

Das zweite zylinderförmige Abstandselement 29 ist um die Ausführöffnung 16 an der Heckseite 17 der Magneteinheit 12 angeordnet. Insbesondere ist das zweite zylinderförmige Abstandselement 29 an einem Heckgehäuse der Magneteinheit 12 angeordnet. Im vorliegenden Ausführungsbeispiel ist das zweite zylinderförmige Abstandselement 29 mit der Heckseite 17 und/oder dem Heckgehäuse der Magneteinheit 12 verschweißt. Alternativ hierzu kann das zweite zylinderförmige Abstandselement 29 auch mit der Heckseite 17 und/oder dem Heckgehäuse der Magneteinheit 12 verklemmt sein und ein Kontaktbereich zwischen dem zweiten zylinderförmige Abstandselement 29 und der Heckseite 17 und/oder dem Heckgehäuse der Magneteinheit 12 mittels HF-dichter Kontaktfedern HF-dicht gehalten werden.

Die beiden Türeinheiten 28 sind zu einem Öffnen oder Verschließen des Probenaufnahmebereichs 12 ausgebildet. Die beiden Türeinheiten 28 weisen jeweils zwei Klappen 30 und eine Antriebseinheit 31 auf. Die Antriebseinheiten 31 sind im vorliegenden Ausführungsbeispiel jeweils von einer elektrischen Antriebseinheit gebildet, mittels der ein Antriebsmoment für eine Schließbewegung und/oder Öffnungsbewegung der beiden Klappen 30 der Türeinheiten 28 generiert wird. Zudem weisen die beiden Türeinheiten 28 jeweils ein Übertragungselement 32 auf zu einer Übertragung des von der elektrischen Antriebseinheit generierten Antriebsmoment auf die beiden Klappen 30. Das Übertragungselement 32 umfasst dabei einen Übertragungsriemen und/oder eine Kette. In einer alternativen Ausgestaltung können die beiden Antriebseinheiten 31 jeweils als pneumatische Antriebseinheiten mit einem pneumatischen Übertragungselement, beispielweise einem Luftschlauch, ausgebildet sein.

Die erste Klappen 30 und die zweite Klappen 30 sind dabei HF-dicht verschließbar ausgebildet. Hierzu weisen die beiden Türeinheiten 28 jeweils mehrere HF-Dichtungen 33 auf. Diese HF-Dichtungen 33 sind jeweils in Kontaktbereichen 34 zwischen den ersten Klappen 30 und den zweiten Klappen 30 angeordnet. Zudem ist zumindest eine HF-Dichtung 33 in einem Kontaktbereich 34 zwischen den ersten Klappen 30 und den zylinderförmigen Abstandselementen 29 angeordnet. Zudem ist zumindest eine HF-Dichtung 33 in einem Kontaktbereich 34 zwischen den zweiten Klappen 30 und den zylinderförmigen Abstandselementen 29 angeordnet. Die HF-Dichtungen sind bevorzugt als Kontaktfedern, insbesondere elektrische leitende Kontaktfedern, ausgebildet. Bevorzugt sind die HF-dichten Kontaktfedern elektrisch leitend ausgebildet, so dass ein elektrischer Kontakt zwischen den beiden Klappen 30 und/oder zwischen den ersten Klappen 30 und dem zylinderförmigen Abstandselementen 29 und/oder den zweiten Klappen und den zylinderförmigen Abstandselementen 29 für eine HF-Schirmung vorhanden ist.

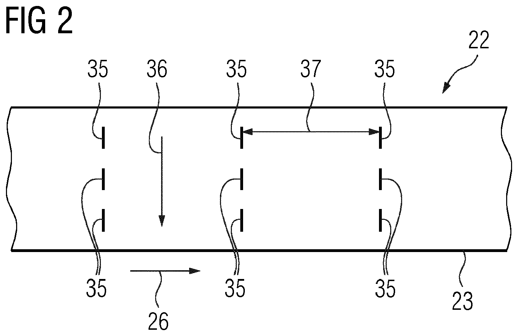

Für ein HF-dichtes Schließen der ersten Klappen 30 mit den zweiten Klappen 30 weist das Transportband 23 der Transportvorrichtung 22 Aussparungen 35 auf (Fig. 2). Die Aussparungen 35 erstrecken sich in Querrichtung 36 des Transportbands 23, wobei die Querrichtung 36 im Wesentlichen quer zur Längsrichtung des Transportbands 23 und/oder quer zur Transportrichtung 26 des Transportbands 23 ausgerichtet ist, in gleichmäßigen Abständen. Insbesondere sind die Aussparungen 35 in Querrichtung 36 in einer Linie nacheinander angeordnet. Zudem weisen auch die ersten Klappen 30 jeweils eine Verzahnung auf, die korrespondierend zu den Aussparungen 35 des Transportbands 23 in Querrichtung 36 ausgebildet ist. Weiterhin weisen auch die zweiten Klappen 30 eine Verzahnung auf, die korrespondierend zur Verzahnung der ersten Klappen 30 ausgebildet ist.

In einem geschlossenen Zustand der jeweiligen Türeinheit 28 sind die ersten Klappen 30 und/oder die zweiten Klappen 30 zumindest teilweise innerhalb den Aussparungen 35 des Transportbands 23 angeordnet. Dabei greifen die Verzahnungen der ersten Klappen 30 in die Verzahnungen der zweiten Klappen 30. Dabei wird in dem geschlossenen Zustand der beiden Türeinheiten 28 ein Kontakt, insbesondere ein mechanischen Kontakt und ein elektrisch leitender Kontakt, zwischen den beiden Klappen 30 hergestellt. Dabei ist ein Kontaktbereich 34 zwischen den beiden Klappen 30 in den Aussparungen 35 des Transportbands 23 angeordnet. Durch den elektrischen leitenden Kontakt zwischen den beiden Klappen 30 wird ein HF-dichtes Schließen der Türeinheiten 28 erreicht.

Weiterhin weist das Transportband 23 mehrere in Längsrichtung und/oder Transportrichtung 26 nacheinander angeordnete Aussparungen 35 auf (Fig. 2). Dabei sind die in Längsrichtung und/oder Transportrichtung 26 nacheinander angeordneten Aussparungen 35 in gleichmäßigen Abständen 37 zueinander am Transportband 23 angeordnet. Bevorzugt sind zwischen den Aussparungen 35 in Längsrichtung und/oder Transportrichtung 26 jeweils Lagerbereiche zur Lagerung der Proben 11 angeordnet. Bei den in Längsrichtung und/oder Transportrichtung 26 nacheinander angeordneten Aussparungen 35 wiederholt sich dabei das Muster der Aussparungen 35 in Querrichtung 36 des Transportbands 23, so dass nach jeder Probe 11 ein Schließen der Türeinheiten 28 möglich ist.

Des Weiteren weist die Magnetresonanzvorrichtung 10 eine Steuereinheit 38 auf (Fig. 1). Mittels der Steuereinheit 38 erfolgt eine zentrale Steuerung der Magnetresonanzvorrichtung 10. Beispielsweise wird mittels der Steuereinheit 38 ein Ausspielen eines Hochfrequenzpulses mittels der Hochfrequenzantenneneinheit 20 gesteuert. Zudem wird mittels der Steuereinheit 38 auch ein Erfassen von Magnetresonanzdaten und/oder ein Auswerten der erfassten Magnetresonanzdaten gesteuert. Hierzu kann die Steuereinheit weitere Einheiten wie beispielsweise Auswerteeinheiten usw. umfassen.

Des Weiteren ist die Steuereinheit 38 dazu ausgebildet, eine Bewegung der Transportvorrichtung 22, insbesondere des Transportbands 23, und/oder ein Schließen der zweit Türeinheiten 28 automatisch an Magnetresonanzmessungen von aufeinander folgenden Proben 11 anzupassen. Dabei werden von der Steuereinheit 38 nach einer Magnetresonanzmessung die beiden Türeinheiten 28 derart angesteuert werden, dass diese die Klappen 30 öffnen. Nach dem Öffnen der Klappen 30 wird die Transportvorrichtung 22 derart angesteuert, dass das Transportband 23 so weit verfährt, bis die nächste Probe 11 im FOV 24 der Magneteinheit 12 angeordnet ist. Anschließend werden von der Steuereinheit 38 die beiden Türeinheiten 28 derart angesteuert, dass diese die Klappen 30 wieder schließen. Anschließend erfolgt die Magnetresonanzmessung der Probe 11, gesteuert von der Steuereinheit 38.

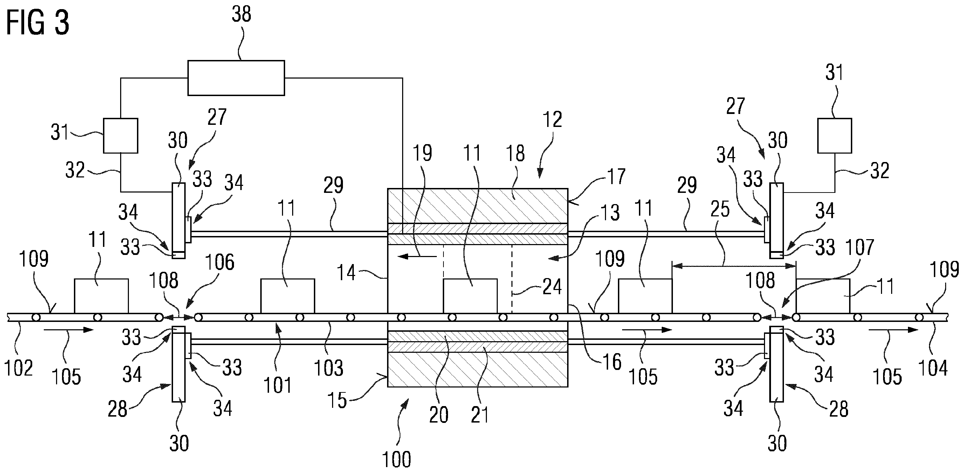

In Fig. 3 ist ein alternatives Ausführungsbeispiel einer Magnetresonanzvorrichtung 100 dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu dem Ausführungsbeispiel in Fig. 1 und 2, wobei bezüglich gleichbleibender Bauteile, Merkmale und Funktionen auf die Beschreibung des Ausführungsbeispiels in Fig. 1 und 2 verwiesen wird.

Die Magnetresonanzvorrichtung 100 in Fig. 3 unterscheidet sich von der Magnetresonanzvorrichtung 10 aus Fig. 1 in einer Ausgestaltung der Transportvorrichtung 101. Die Transportvorrichtung 101 weist drei Transportbänder 102, 103, 104 auf, die in Transportrichtung 105 nacheinander angeordnet sind. Ein erstes Transportband 102 erstreckt sich dabei in Transportrichtung 105 bis zur ersten Türeinheit 28. Ein zweites Transportband 103 erstreckt sich in Transportrichtung 105 von der ersten Türeinheit 28 bis an die zweite Türeinheit 28. Ein drittes Transportband 104 erstreckt sich in Transportrichtung 105 von der zweiten Türeinheit 28 weg. Dabei weisen jeweils zwei benachbarte Transportbänder 102, 103, 104 einen Übergangsbereich 106, 107 auf. Insbesondere ist zwischen dem ersten Transportband 102 und dem zweiten Transportband 103 ein erster Übergangsbereich 106 angeordnet und zwischen dem zweiten Transportband 103 und dem dritten Transportband 104 ein zweiter Übergangsbereich 107 angeordnet.

Die beiden Übergangsbereiche 106, 107 weisen dabei in Transportrichtung 105 eine Länge 108 auf, die im Wesentlichen einer Dicke der Türeinheiten 28 entspricht. Dabei ist in dem ersten Übergangsbereich 106 die erste Türeinheit 28 angeordnet und in dem zweiten Übergangsbereich 107 die zweite Türeinheit 28. In einem geschlossenen Zustand der ersten Türeinheit 28 befinden sich somit die erste Klappe 30 und die zweite Klappe 30 der ersten Türeinheit 28 in dem ersten Übergangsbereich 106 und/oder zwischen dem ersten Transportband 102 und dem zweiten Transportband 103. Ebenso befinden sich in einem geschlossenen Zustand der zweiten Türeinheit 28 somit die erste Klappe 30 und die zweite Klappe 30 der zweiten Türeinheit 28 in dem zweiten Übergangsbereich 107 und/oder zwischen dem zweiten Transportband 103 und dem dritten Transportband 104. Die einzelnen Transportbänder 102, 103, 104 weisen zudem eine durchgehende Lagerfläche 109 zur Lagerung von Proben 11 auf, wobei die Lagerfläche 109 frei von Aussparungen ist.

Eine Ausgestaltung und Anordnung der Magneteinheit 12 entsprechen dabei den Ausführungen zu Fig. 1, auf die hiermit verwiesen wird. Eine Ausgestaltung und Anordnung der zylinderförmigen Abstandselemente 29 entsprechen dabei den Ausführungen zu Fig. 1, auf die hiermit ebenfalls verwiesen wird. Zudem entsprechen auch eine Ausgestaltung und Anordnung der Türeinheiten 28 den Ausführungen zu Fig. 1, auf die hiermit verwiesen wird.

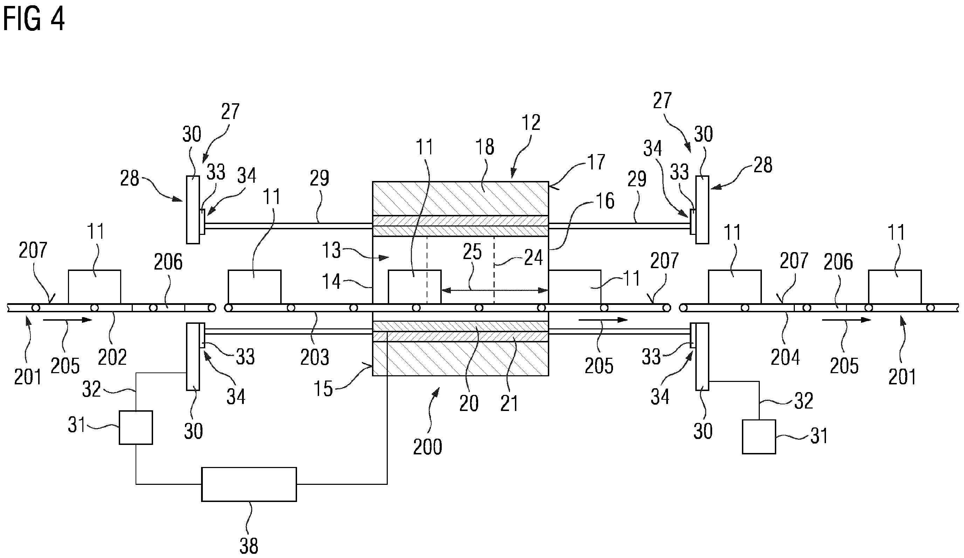

In Fig. 4 ist ein alternatives Ausführungsbeispiel der Magnetresonanzvorrichtung 200 dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu den Ausführungsbeispielen in den Fig. 1 bis 3, wobei bezüglich gleichbleibender Bauteile, Merkmale und Funktionen auf die Beschreibung der Ausführungsbeispiele in den Fig. 1 bis 3 verwiesen wird.

Die Magnetresonanzvorrichtung 100 in Fig. 4 weist eine Transportvorrichtung 201 auf, die drei Transportbänder 202, 203, 204 umfasst. Die drei Transportbänder 202, 203, 204 sind in Transportrichtung 205 nacheinander angeordnet. Ein erstes Transportband 202 erstreckt sich dabei in Transportrichtung 205 bis kurz nach der ersten Türeinheit 28. Ein zweites Transportband 203 erstreckt sich in Transportrichtung 205 von dem ersten Transportband 202, insbesondere von dem in Transportrichtung 205 weisenden Ende des ersten Transportbands 202 bis kurz vor die zweite Türeinheit 28. Ein drittes Transportband 204 erstreckt sich von kurz vor der zweiten Türeinheit 28 in Transportrichtung 205 weg.

Das erste Transportband 202 und das dritte Transportband 204 weisen dabei ein HF-schirmendes Material auf. Insbesondere weisen hierbei das erste Transportband 202 und das dritte Transportband 204 jeweils Segmente 206 mit einem HF-schirmenden Material auf. Bevorzugt sind diese Segmente 206 in gleichmäßigen Abständen in den beiden Transportbändern 202 204 angeordnet und dazwischen jeweils ein Lagerbereich 207 zur Lagerung und/oder Positionierung von Proben 11.

Bei einem Schließen der beiden Türeinheiten 28, insbesondere der ersten Klappen 30 mit dem zweiten Klappen 30, bildet das HF-schirmende Material, insbesondere die Segmente 206 mit dem HF-schirmenden Material, einen Kontaktbereich zwischen den beiden Klappen 30. Bevorzugt weisen hierbei die beiden Klappen 30 der jeweiligen Türeinheit 28 einen glatten, unverzahnten Randbereich auf, der bei einem Schließen der Türeinheit 28 in Kontakt mit dem ersten oder dem dritten Transportband 202, 204 kommt, und damit einen elektrisch leitenden Kontakt zwischen den beiden Klappen 30 erzeugt.

Das zweite Transportband 203 dagegen weist kein HF-schirmendes Material auf, um eine unerwünschte Anziehung durch den Grundmagneten 18 der Magneteinheit 12 und/oder eine Erzeugung von Wirbelströmen zu verhindern.

Eine Ausgestaltung und Anordnung der Magneteinheit 12 entsprechen dabei den Ausführungen zu Fig. 1, auf die hiermit verwiesen wird. Eine Ausgestaltung und Anordnung der zylinderförmigen Abstandselemente 29 entsprechen dabei den Ausführungen zu Fig. 1, auf die hiermit ebenfalls verwiesen wird. Zudem entsprechen auch eine Ausgestaltung und Anordnung der Türeinheiten 28 den Ausführungen zu Fig. 1, auf die hiermit verwiesen wird.

Die in den Fig. 1 bis 4 dargestellten Magnetresonanzvorrichtungen 10, 100, 200 können selbstverständlich weitere Komponenten umfassen, die Magnetresonanzvorrichtungen 10, 100, 200 gewöhnlich aufweisen. Eine allgemeine Funktionsweise einer Magnetresonanzvorrichtung 10, 100, 200 ist zudem dem Fachmann bekannt, so dass auf eine detaillierte Beschreibung der weiteren Komponenten verzichtet wird.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Magnetresonanzvorrichtung, insbesondere zum Erfassen von zumindest einer Eigenschaft einer Probe, vorzugsweise eines Lebensmittels, umfassend eine Magneteinheit, die einen Grundmagneten und eine Hochfrequenzantenneneinheit umfasst, einen Probenaufnahmebereich, wobei der Probenaufnahmebereich zumindest teilweise von der Magneteinheit umgeben ist, und eine Transportvorrichtung zu einem Einbringen der Probe in den Probenaufnahmebereich,
**gekennzeichnet durch** eine Hochfrequenzabschirmeinheit, die den Probenaufnahmebereich nach außen abschirmt.

2. Magnetresonanzvorrichtung nach Anspruch 1,
**dadurch gekennzeichnet, dass** der Probenaufnahmebereich zylinderförmig von der Magneteinheit umgeben ist, eine Einführöffnung und eine Ausführöffnung aufweist, wobei die Hochfrequenzabschirmeinheit um die Einführöffnung und/oder um die Ausführöffnung angeordnet ist.

3. Magnetresonanzvorrichtung nach einem der vorhergehenden Ansprüche,
**dadurch gekennzeichnet, dass** die Hochfrequenzabschirmeinheit außerhalb des Probenaufnahmebereichs angeordnet ist.

4. Magnetresonanzvorrichtung nach einem der vorhergehenden Ansprüche,
**dadurch gekennzeichnet, dass** die Hochfrequenzabschirmeinheit zumindest eine Türeinheit aufweist, wobei mittels der zumindest einen Türeinheit der Probenaufnahmebereich verschließbar oder öffenbar ausgebildet ist.

5. Magnetresonanzvorrichtung nach Anspruch 4,
**dadurch gekennzeichnet, dass** die Hochfrequenzabschirmeinheit zumindest ein zylinderförmiges Abstandselement aufweist, das an einer Frontseite und/oder an einer Heckseite der Magneteinheit angeordnet ist, wobei das zumindest eine Abstandselement zwischen dem Probenaufnahmebereich und der zumindest einen Türeinheit angeordnet ist.

6. Magnetresonanzvorrichtung nach Anspruch 5,
**dadurch gekennzeichnet, dass** das zumindest eine zylinderförmige Abstandselement HF-dicht an der Frontseite und/oder an der Heckseite der Magneteinheit angeordnet ist.

7. Magnetresonanzvorrichtung nach einem der Ansprüche 4 bis 6,
**dadurch gekennzeichnet, dass** die zumindest eine Türeinheit zumindest eine Klappe aufweist und die Hochfrequenzabschirmeinheit zumindest eine elektrische Antriebseinheit und/oder zumindest eine pneumatische Antriebseinheit aufweist, wobei mittels der zumindest einen elektrischen Antriebseinheit und/oder der zumindest einen pneumatischen Antriebseinheit ein Antriebsmoment für eine Bewegung der zumindest einen Klappe der zumindest einen Türeinheit generierbar ist.

8. Magnetresonanzvorrichtung nach Anspruch 7,
**dadurch gekennzeichnet, dass** die Hochfrequenzabschirmeinheit zumindest ein Übertragungselement aufweist, um ein von der zumindest einen elektrischen Antriebseinheit und/oder der zumindest einen pneumatischen Antriebseinheit generiertes Antriebsmoment auf die zumindest eine Klappe der zumindest einen Türeinheit zu übertragen.

9. Magnetresonanzvorrichtung nach einem der Ansprüche 4 bis 8,
**dadurch gekennzeichnet, dass** die zumindest eine Türeinheit eine erste Klappe und eine zweite Klappe umfasst, wobei die erste Klappe und die zweite Klappe HF-dicht verschließbar sind.

10. Magnetresonanzvorrichtung nach einem der Ansprüche 4 bis 9,
**dadurch gekennzeichnet, dass** die zumindest eine Türeinheit zumindest eine HF-Dichtung aufweist, wobei die zumindest eine HF-Dichtung in einem Kontaktbereich zwischen der ersten Klappe und der zweiten Klappe und/oder in einem Kontaktbereich zwischen der ersten Klappe und dem zumindest einen zylinderförmigen Abstandselement und/oder in einem Kontaktbereich zwischen der zweiten Klappe und dem zumindest einen zylinderförmigen Abstandselement angeordnet ist.

11. Magnetresonanzvorrichtung nach einem der Ansprüche 9 bis 10,
**dadurch gekennzeichnet, dass** die Transportvorrichtung zumindest ein Transportband aufweist, wobei das zumindest eine Transportband zumindest eine Aussparung aufweist, wobei die erste Klappe und/oder die zweite Klappe in einem geschlossenen Zustand der zumindest einen Türeinheit zumindest teilweise innerhalb der zumindest einen Aussparung angeordnet sind.

12. Magnetresonanzvorrichtung nach einem der Ansprüche 9 bis 11,
**dadurch gekennzeichnet, dass** das zumindest eine Transportband mehrere Aussparungen aufweist, die in Querrichtung des Transportbands in einer Linie nacheinander angeordnet sind, wobei die erste Klappe in einem Kontaktbereich mit der zweiten Klappe eine Verzahnung aufweist und die zweite Klappe in dem Kontaktbereich mit der ersten Klappe eine zur ersten Klappe korrespondierende Verzahnung aufweist, wobei in einem geschlossenem Zustand der Türeinheit die beiden Verzahnungen durch die Aussparungen des Transportband ineinander greifen.

13. Magnetresonanzvorrichtung nach einem der Ansprüche 11 bis 12,
**dadurch gekennzeichnet, dass** das zumindest eine Transportband mehrere in Längsrichtung nacheinander angeordnete Aussparungen aufweist.

14. Magnetresonanzvorrichtung nach einem der Ansprüche 4 bis 10 und einem der Ansprüche 11 bis 13,
**dadurch gekennzeichnet, dass** die Transportvorrichtung zumindest zwei Transportbänder, die nacheinander in Transportrichtung angeordnet sind, und zumindest einen Übergangsbereich zwischen den zumindest zwei Transportbändern aufweist, wobei in diesem zumindest eine Übergangsbereich die zumindest eine Türeinheit in einem geschlossenen Zustand der Türeinheit angeordnet ist.

15. Magnetresonanzvorrichtung nach einem der vorhergehenden Ansprüche,
**dadurch gekennzeichnet, dass** die Transportvorrichtung zumindest ein Transportband aufweist, wobei das zumindest eine Transportband ein HF-schirmendes Material umfasst.

16. Magnetresonanzvorrichtung nach einem der vorhergehenden Ansprüche,
**gekennzeichnet durch** eine Steuereinheit, wobei die Steuereinheit dazu ausgebildet ist, eine Bewegung der Transportvorrichtung und/oder ein Schließen der zumindest einen Türeinheit automatisch an Magnetresonanzmessungen von aufeinander folgenden Proben anzupassen.
